# FASCICULE DE BREVET EUROPEEN

(11) **EP 3 770 152 B1**
(45) Date de publication et mention de la délivrance du brevet: **04.03.2026**
(21) Numéro de dépôt: 20186798.3
(22) Date de dépôt: 20.07.2020
(51) Int. Cl.: C07D 311/04, C07D 413/04

(54) **PROCÉDÉ DE FABRICATION DE CHROMENES PAR CATALYSE A L'OR DESTINÉS À LA PRÉPARATION DE RESINES THERMODURCISSABLES**
VERFAHREN ZUR HERSTELLUNG VON CHROMENEN DURCH GOLDKATALYSE, DIE FÜR DIE HERSTELLUNG VON WÄRMEHÄRTBAREN HARZEN BESTIMMT SIND
METHOD FOR MANUFACTURING CHROMENES BY GOLD-BASED CATALYSIS INTENDED FOR THE PREPARATION OF HEAT-SETTING RESINS

(30) Priorité: 23.07.2019 FR 1908325
(43) Date de publication de la demande: 27.01.2021
(73) Titulaire: ArianeGroup SAS, 78130 Les Mureaux (FR); CENTRE NATIONAL DE LA RECHERCHE SCIENTIFIQUE (C.N.R.S.), 75016 Paris (FR); Université De Reims Champagne-Ardenne, 51100 Reims (FR)
(72) Inventeur: POUYET, Robin, 63800 COURNON (FR); COQUERET, Xavier, 51100 REIMS (FR); DEFOORT, Brigitte, 33160 Saint-Médard-en-Jalles (FR); RIVIERES, Bastien, 81640 LE SEGUR (FR)
(74) Mandataire: Cabinet Beau de Loménie

(56) Documents cités:
- EP-A2- 0 350 747
- WO-A1-2017/129661
- RAJEEV S. MENON ET AL.: "The AU(I)-catalyzed Intramolecular hydroarylation of terminal alkynes under mild conditions: Application to the synthesis of 2H-Chromenes, Coumarins, Benzofurans, and Dihydroquinolines", J. ORG. CHEM., vol. 74, 2009, pages 8901 - 8903, XP055082760
- ANTONIO ARCADI ET AL: "Gold versus silver catalyzed intramolecular hydroarylation reactions of [(3-arylprop-2-ynyl)oxy]benzene derivatives", ORGANIC & BIOMOLECULAR CHEMISTRY, vol. 10, no. 48, 1 January 2012 (2012-01-01), pages 9700, XP055700971, ISSN: 1477-0520, DOI: 10.1039/c2ob26763b
- WEI FANG ET AL: ")-catalyzed intramolecular hydroarylation and the subsequent ring enlargement of methylenecyclopropanes to cyclobutenes", RSC ADVANCES, vol. 6, no. 46, 1 January 2016 (2016-01-01), pages 40474 - 40479, XP055701069, DOI: 10.1039/C6RA02549H

## Description

La présente invention concerne le domaine des résines thermodurcissables, et des matériaux obtenus à partir de ces résines et en particulier leurs procédés de fabrication. Ces résines visent à remplacer les résines phénoliques dans toutes les applications dans lesquelles celles-ci sont utilisées, et notamment les matériaux dits « ablatifs ».

Un matériau ablatif est défini comme un matériau qui est capable de subir une ablation, c'est-à-dire une perte de substance par décomposition chimique, changement d'état ou érosion mécanique sous l'effet d'un flux de matière ou de rayonnement (Journal Officiel de la République Française du 22 septembre 2000). C'est en particulier le cas des matériaux qui entrent dans la constitution des boucliers thermiques destinés à l'aérospatiale et des parois de tuyères des moteurs à propulsions. Typiquement, dans ce cas, la couche externe du matériau ablatif qui est directement en contact avec l'environnement, subit sous l'effet de la chaleur une transformation chimique, ainsi qu'une récession liée à cette transformation. Cette couche externe rayonne donc vers l'extérieur et sa transformation chimique consomme de l'énergie. Ces deux effets contribuent à une moindre transmission de la chaleur vers les couches internes du matériau et donc à une isolation thermique de la structure sous-jacente. Un bon matériau ablatif doit être tel que sa transformation chimique sous l'effet de la chaleur soit endothermique, sa conductivité thermique soit faible en régime stationnaire et/ou en transitoire et sa transformation chimique ne s'accompagne pas d'une récession trop rapide. En particulier, pour remplir ce dernier point, il faut que la transformation chimique du matériau ablatif s'accompagne de la formation d'une croûte à base de carbone ou silice provenant de la pyrolyse de la résine.

Ceci est en particulier obtenu pour des résines ayant un taux de coke élevé. Le taux de coke est défini comme la masse de résidu qui est obtenu quand on décompose un échantillon d'un polymère organique par pyrolyse, à une température de 900°C sous atmosphère inerte (azote ou argon), rapportée à la masse initiale de cet échantillon. Les résines les plus intéressantes présentent un taux de coke supérieure à 50%.

Les résines phénoliques présentent en général un tel taux de coke et sont obtenues par polycondensation de monomères issus de la pétrochimie : le phénol et le formaldéhyde, ce qui leur vaut d'être aussi appelées résines phénol-formaldéhydes ou résines formophénoliques. Les précurseurs des résines phénoliques, le phénol et le formaldéhyde, sont respectivement CMR 2 et 1B. Ces deux composés sont donc sous la surveillance du Règlement (CE) n° 1907/2006 du Parlement Européen (REACH) qui vise à mieux protéger la santé humaine et l'environnement contre les risques liés aux substances chimiques. Il s'avère, de plus, que la polycondensation du phénol et du formaldéhyde n'est jamais achevée, d'où la présence de composés volatils et de molécules d'eau qui sont très difficiles à éliminer si un cycle thermique bien défini n'est pas suivi au cours de cette polycondensation et qui peuvent conduire à des matériaux poreux dans leur état natif ainsi qu'à des dégazages durant la vie des matériaux fabriqués à partir de résines phénoliques. Or, ces dégazages peuvent avoir des conséquences très néfastes dans certaines applications comme, par exemple, les applications aérospatiales.

Compte-tenu de la place qu'occupent actuellement les résines phénoliques dans l'industrie des matériaux plastiques et de ses inconvénients, de nouvelles résines thermodurcissables présentant des propriétés similaires à celles des résines phénoliques ont été obtenues à partir de précurseurs différents. Ainsi la demande de brevet WO2017/129661 décrit de telles résines et leurs procédés de fabrication. De telles résines ont un taux de coke supérieure à 50% et peuvent donc être utilisées comme matériaux ablatifs. Les précurseurs utilisés sont en particulier des molécules aromatiques porteuses de fonctions éther de propargyle. Toutefois l'énergie trop importante libérée pendant leur polymérisation pourrait engendrer un emballement thermique lors de la fabrication des matériaux composites. Ainsi, afin d'obtenir une enthalpie de polymérisation d'environ 800-900 J/g avec une perte de masse la plus faible possible lors de la polymérisation, il est nécessaire dans le procédé décrit dans cette demande de maintenir un traitement thermique long pendant la polymérisation afin de prévenir tout emballement thermique. Cette solution n'est donc pas optimisée vis-à-vis de la fabrication des pièces épaisses pouvant mesurer jusqu'à plusieurs dizaines de millimètres d'épaisseurs.

Les inventeurs se sont rendus compte qu'il était possible de diminuer l'énergie libérée pendant la polymérisation des résines à terminaisons éther de propargyle d'un facteur 6 par la conversion de la fonction éther de propargyle en fonction chromène par catalyse homogène à l'or et ainsi d'abaisser l'enthalpie de polymérisation jusqu'à une valeur < 500 J/g.

L'article de Rajeev S. Menon et al. (J. Org. Chem. 2009, 74, pages 8901-8903) décrit un procédé de conversion de la fonction éther de propargyle en fonction chromène par catalyse homogène à l'or à l'aide du catalyseur (acétonitrile)[(2-biphényl)di-tert-butylphosphine]or(I) hexafluoroantimonate de certains composés aromatiques porteurs de fonctions éther de propargyle tel que le catéchol propargylé. Toutefois ce procédé n'a pas été appliqué au résorcinol propargylé, à l'eugénol propargylé, à l'eugénol couplé propargylé, à l'isoeugénol couplé propargylé ou à l'isoeugénol propargylé.

La demande EP0350747 décrit un procédé de conversion d'éthers de propargyle aromatique en chromène par catalyse aux sels de cuivre ou de zinc et sa polymérisation.

La présente invention concerne donc un procédé de préparation d'un matériau en résine thermodurcie selon la revendication 1. Ledit procédé comprend l'étape (a) de transformation d'un éther de propargyle aromatique de formule générale (I) suivante 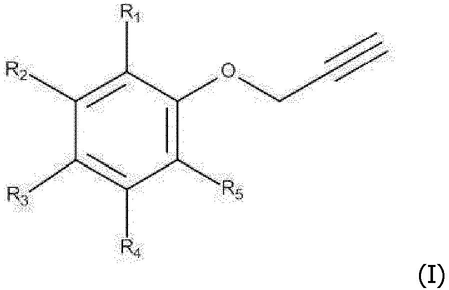 dans laquelle :
**R₁ et R₅** représentent indépendamment l'un de l'autre un atome d'hydrogène, un groupe alcène en C₂-C₆, alcyne en C₂-C₆, O-alkyle en C₁-C₆, O-alcène en C₂-C₆ ou O-alcyne en C₂-C₆, à la condition que l'un au moins des **R₁ et R₅** représente un atome d'hydrogène et que les groupes **R₁ et R₅** ne représentent pas un groupe O-propargyl;
**R₂ et R₄** représentent indépendamment l'un de l'autre un atome d'hydrogène, un groupe alcène en C₂-C₆, alcyne en C₂-C₆ tel qu'un propargyle, O-alkyle en C₁-C₆, O-alcène en C₂-C₆ ou O-alcyne en C₂-C₆ tel qu'un O-propargyle ;
et **R₃** représente un atome d'hydrogène ou un groupe alcène en C₂-C₆, le groupe alcène étant éventuellement substitué par un groupe de formule générale (II) suivante 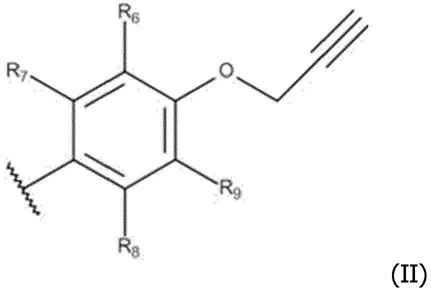 dans laquelle :
   **R₆ et R₉** représentent indépendamment l'un de l'autre un atome d'hydrogène, un groupe alcène en C₂-C₆, alcyne en C₂-C₆, O-alkyle en C₁-C₆, O-alcène en C₂-C₆ ou O-alcyne en C₂-C₆, à la condition que l'un au moins des **R₆ et R₉** représente un atome d'hydrogène ;
   et **R₇ et R₈** représentent indépendamment l'un de l'autre un atome d'hydrogène, un groupe alcène en C₂-C₆, alcyne en C₂-C₆ tel qu'un propargyle, O-alkyle en C₁-C₆, O-alcène en C₂-C₆ ou O-alcyne en C₂-C₆ tel qu'un O-propargyle ;
   à la condition que l'un au moins des **R₁, R₂, R₃, R₄ et R₅** ne représente pas un atome d'hydrogène ou un groupe O-alkyle en C₁-C₆ ;
   et ses isomères cis/trans et ses isomères optiques et leurs mélanges racémiques
   en un chromène par catalyse homogène à l'or (I) avec le catalyseur (acétonitrile)[(2-biphényl)di-tert-butylphosphine]or(I) hexafluoroantimonate dans un solvant organique sous atmosphère inerte ou non inerte.

Au sens de la présente invention on entend par « groupe alcène en C₂-C₆ » tout groupe alcène de 2 à 6 atomes de carbones, linéaire ou ramifié, en particulier le groupe vinyle, le groupe allyle ou le groupe but-2-ényle.

Au sens de la présente invention on entend par « groupe alcyne en C₂-C₆ » tout groupe alcyne de 2 à 6 atomes de carbones, linéaire ou ramifié, en particulier le groupe éthynyle ou le groupe propargyle.

Au sens de la présente invention on entend par « groupe O-alkyle en C₁-C₆ » tout groupe O-alkyle de 1 à 6 atomes de carbones, linéaire ou ramifié, en particulier, le groupe méthoxy ou éthoxy.

Au sens de la présente invention on entend par « groupe O-alcène en C₂-C₆ » tout groupe O-alcène de 2 à 6 atomes de carbones, linéaire ou ramifié.

Au sens de la présente invention on entend par « groupe O-alcyne en C₂-C₆ » tout groupe O-alcyne de 2 à 6 atomes de carbones, linéaire ou ramifié en particulier le groupe O-propargyle.

Avantageusement l'éther de propargyle aromatique de formule générale (I) est choisi dans le groupe constitué par le résorcinol propargylé, l'eugénol propargylé, l'eugénol couplé propargylé, l'isoeugénol couplé propargylé, l'isoeugénol propargylé et leurs mélanges et leurs isomères cis/trans et leurs isomères optiques et leurs mélanges racémiques, plus avantageusement il s'agit du résorcinol propargylé. Ces produits sont bien connus de l'homme du métier et peuvent être préparés par des procédés bien connus, tels que ceux décrits dans la demande WO2017/129661. Ils ont l'avantage de pouvoir être issus de composés biosourcés tels que le résorcinol, l'eugénol, l'eugénol couplé, l'isoeugénol et l'isoeugénol couplé.

Le résorcinol propargylé a ainsi la formule générale suivante :

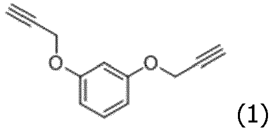

L'eugénol propargylé a ainsi la formule générale suivante :

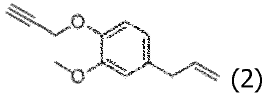

L'eugénol couplé propargylé a ainsi la formule générale suivante : 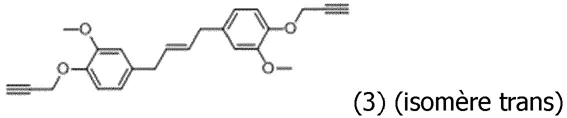 ou la formule générale suivante 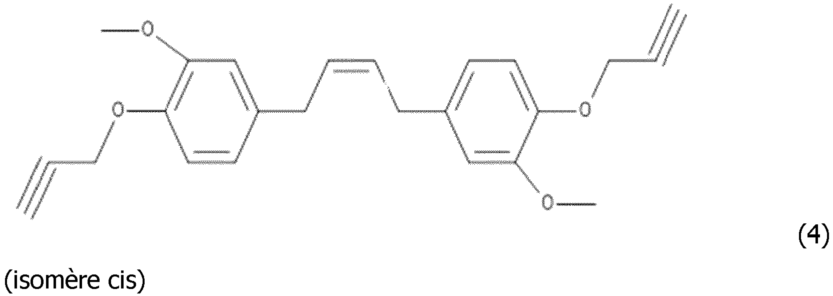 ou un mélange de ces deux isomères.

L'isoeugénol propargylé a ainsi la formule générale suivante :

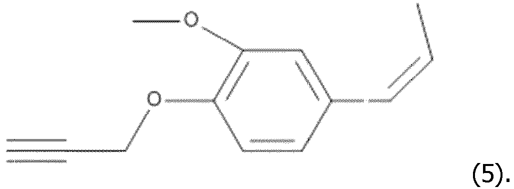

L'isoeugénol couplé propargylé a ainsi la formule générale suivante : 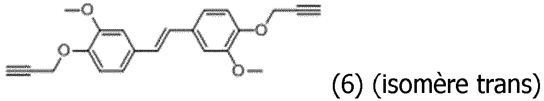 ou la formule générale suivante 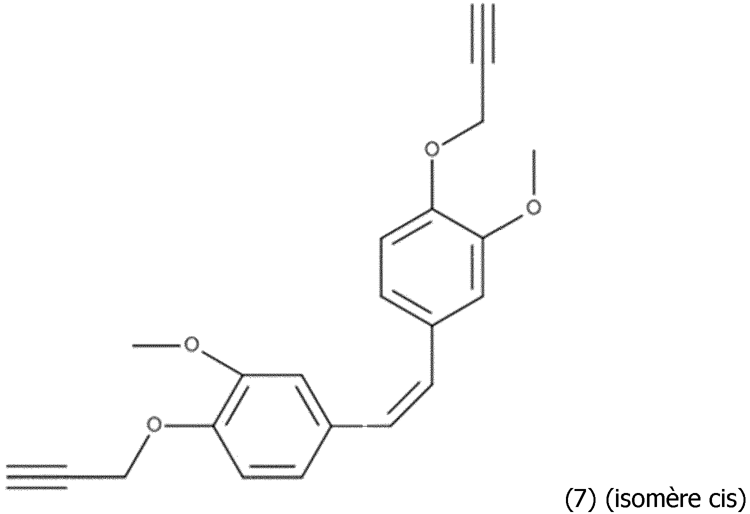 ou un mélange de ces deux isomères.

Le solvant utilisé dans l'étape a) selon l'invention est un solvant organique. Il peut ainsi s'agir du dichlorométhane, du tétrahydrofurane, du 2-méthyltétrahydrofurane ou de leurs mélanges. Avantageusement, il s'agit d'un solvant non chloré tel que tétrahydrofurane, le 2-méthyltétrahydrofurane ou leurs mélanges. Ainsi, en particulier le solvant polaire est choisi parmi le tétrahydrofurane et le 2-méthyltétrahydrofurane.

Le catalyseur de l'étape a) selon la présente invention est donc l'(acétonitrile)[(2-biphényl)di-tert-butylphosphine]or(I) hexafluoroantimonate de formule générale 8 suivante :

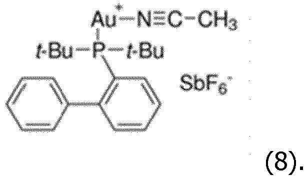

Avantageusement, la teneur en catalyseur dans le milieu réactionnel est comprise entre 0,01 et 2% molaire, avantageusement entre 0,1 et 0,5 % molaire.

Le catalyseur doit être éliminé à la fin de l'étape a). Le procédé comprend donc une étape d'élimination du catalyseur du milieu réactionnel, avantageusement par des procédés bien connus de l'homme du métier.

Par exemple, dans le cas où le solvant est du dichlorométhane, cette étape d'élimination peut consister en une simple filtration sur couche de silice. Dans le cas où le solvant est un solvant non chloré tel que le tétrahydrofurane ou le 2-méthyltétrahydrofurane, cette étape est plus difficile à mettre en œuvre. Toutefois, il est possible d'utiliser dans ce cas une colonne chromatographique avec un éluant éther de pétrole/acétate d'éthyle en particulier en proportion en volume 9/1, après évaporation du solvant. Ainsi avantageusement, le procédé selon la présente invention comprend une étape supplémentaire d'élimination du solvant, en particulier par évaporation, avant l'étape d'élimination du catalyseur. Cette étape d'élimination du solvant peut avoir lieu également après l'élimination du catalyseur.

De façon avantageuse, l'étape a) selon la présente invention est mis en œuvre à une température comprise entre 0 et 50°C, en particulier entre 20 et 50°C.

L'étape a) selon l'invention peut avoir lieu sous atmosphère inerte (azote ou argon par exemple) ou non.

La durée de réaction dépend des conditions réactionnelles telles que les éthers de propargyles aromatiques utilisés, la température de réaction, la teneur en catalyseur, le solvant polaire utilisé et peut ainsi varier de quelques secondes (par exemple 30 s) à plusieurs heures (par exemple 5 heures).

Les inventeurs se sont aperçus qu'il n'était pas nécessaire d'avoir une conversion quantitative des fonctions éthers de propargyle en chromène pour obtenir une enthalpie de polymérisation inférieure à 500J/g. En effet l'énergie libérée pendant la polymérisation pour un substrat donné est dépendant de sa masse molaire et de sa fonctionnalité. En utilisant l'énergie libérée par fonction propargyle et par fonction chromène, combiné à la masse molaire de chaque substrat, il est possible de déterminer théoriquement le pourcentage de fonctions propargyle résiduelles maximum afin de se trouver en dessous des 500 J/g. ces valeurs ont été comparés aux valeurs expérimentales obtenues et sont similaires. Ainsi le tableau 1 ci-après indique le pourcentage de fonctions propargyle résiduelles théorique afin de se trouver en dessous des 500 J/g d'enthalpie de réaction lors de la polymérisation. Le pourcentage de fonctions propargyles résiduelles théorique est calculé de la façon suivante : (nombre de moles de fonctions propargyles à l'issue de la réaction) / (nombre de moles de fonctions propargyle avant le début de la réaction) x100.

**Tableau 1**

| Substrat | M (g/mol) | Fonctionnalité en groupements propargyle | Pourcentage molaire de fonctions propargyle résiduelles théorique pour atteindre une enthalpie de 500 J/g |
|---|---|---|---|
| Résorcinol propargylé | 186,21 | 2 | 11 |
| Eugénol couplé propargylé | 376,44 | 2 | 39 |
| Isoeugénol couplé propargylé | 348,39 | 2 | 35 |

Ainsi avantageusement, la conversion des éthers de propargyles aromatiques en chromène par l'étape a) selon la présente invention n'est pas totale et le chromène obtenu comprend des fonctions propargyles résiduelles. De façon avantageuse, le pourcentage molaire de fonctions propargyles résiduelles du chromène est inférieur à 11% lorsque l'éther de propargyle aromatique de formule générale (I) est le résorcinol propargylé, le pourcentage molaire de fonctions propargyles résiduelles du chromène est inférieur à 39% lorsque l'éther de propargyle aromatique de formule générale (I) est l'eugénol couplé propargylé et le pourcentage molaire de fonctions propargyles résiduelles du chromène est inférieur à 35% lorsque l'éther de propargyle aromatique de formule générale (I) est l'isoeugénol couplé propargylé.

Dans le cas où l'éther de propargyle aromatique de formule générale (I) est le résorcinol propargylé, le chromène obtenu à l'aide de l'étape a) selon l'invention peut avoir la formule C et / ou D suivante, avantageusement il s'agit d'un mélange des formules C et D.

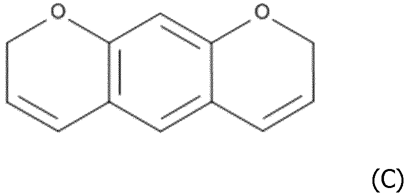

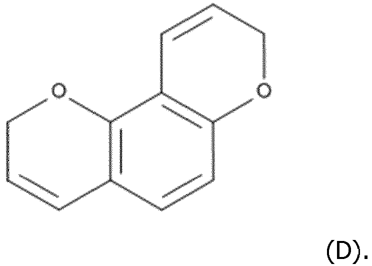

En particulier la proportion molaire de chromène de formule C dans le mélange est comprise entre 50 et 55% et celle de chromène de formule D entre 45 et 50%, selon le solvant polaire utilisé. Ainsi, dans le cas où le dichlorométhane ou le 2-méthyltétrahydrofurane est utilisé, le mélange comprendra 55% de chromène de formule C et 45% de chromène de formule D et dans le cas où le tétrahydrofurane est utilisé, le mélange comprendra 50% de chromène de formule C et 50% de chromène de formule D.

Dans le cas où l'éther de propargyle aromatique de formule générale (I) est le résorcinol propargylé, le chromène obtenu à l'aide de l'étape a) selon l'invention peut également avoir la formule A et / ou B suivante :

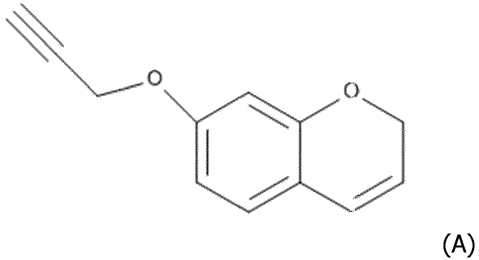

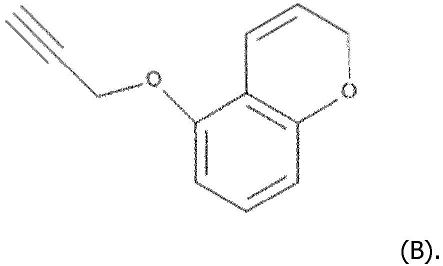

Toutefois ces molécules sont en général rapidement convertis en composés de formule C et D.

L'étape a) selon la présente invention peut également entrainer la formation d'un coproduit du type 2-méthylbenzofurane (impureté), en particulier en une proportion molaire par rapport à la totalité des espèces présentes dans le milieu réactionnel comprise entre 0,5% et 25%, en particulier dans le cas où l'éther de propargyle aromatique de formule générale (I) est le résorcinol propargylé, en une proportion molaire comprise entre 0,5 et 10%. Dans un mode de réalisation avantageux, le procédé selon la présente invention comprend une étape supplémentaire de purification du milieu réactionnel de façon à supprimer cette impureté, avantageusement après l'éventuelle étape de suppression du catalyseur et après l'éventuelle étape d'élimination du solvant.

Avantageusement, le rendement molaire de la réaction de conversion de l'éther de propargyle aromatique en chromène est compris entre 10 et 99%, plus avantageusement entre 50 et 99%, encore plus avantageusement entre 60 et 99%.

Le procédé de préparation d'un matériau en résine thermodurcie selon l'invention comprend les étapes successives suivantes :
- a) transformation d'un éther de propargyle aromatique selon l'étape (a) de la présente invention telle que décrite ci-dessus ;
- b) polymérisation du produit de la réaction obtenu à l'étape a) de façon à obtenir le matériau en résine thermodurcie ;
- c) récupération du matériau en résine thermodurcie obtenu à l'étape b).

Conformément à l'invention, la polymérisation de la résine peut être réalisée par tout moyen susceptible d'induire la polymérisation/réticulation du chromène et, notamment, par application d'un traitement thermique ou d'un traitement lumineux (lumière visible, UV ou IR).

En particulier l'étape b) est mise en œuvre par traitement thermique, avantageusement à une température comprise entre 80°C et 180°C, plus avantageusement à l'aide de plusieurs paliers de chauffe (en particulier 3 ou 4 ou 5), sans ajout d'autres composants, tel que par exemple 2h à 80°C, 2h à 100°C, 2h à 120°C, 2h à 140°C et 2h à 180°C ou 1h à 80°C, 1h à 100°C, 1h à 120°C, 1h à 140°C, 1h à 160°C, 1h à 180°C, 1h à 200°C ou encore 1h à 100°C, 1h30 à 150°C, 3h30 à 210°C et 1h à 200°C ou même encore 1h à 80°C, 2h à 150°C, 2h à 220°C.

Plus particulièrement le procédé selon l'invention peut comprendre entre les étapes b) et c) une étape b1) de recuit à une température supérieure à 200°C mais inférieure à la température de dégradation de la résine, par exemple à 220°C. Cette étape permet d'améliorer les propriétés thermomécaniques de la résine.

Dans un mode de réalisation avantageux, le matériau en résine thermodurcie, est un matériau formant la matrice d'un matériau composite du type comprenant une matrice dans laquelle se trouve un renfort.

Le renfort présent dans le matériau composite peut être de différents types. Ainsi, il peut notamment s'agir d'un renfort constitué de fibres telles que des fibres de verre, des fibres de quartz, des fibres de carbone, des fibres de graphite, des fibres de silice, des fibres métalliques comme des fibres d'acier ou des fibres d'aluminium, des fibres de bore, des fibres céramiques comme des fibres de carbure de silicium ou de carbure de bore, des fibres organiques de synthèse comme des fibres d'aramide, des fibres de polyéthylène, des fibres de polyester ou des fibres de poly(p-phénylène benzobisoxazole), plus connues sous le sigle PBO, des fibres organiques naturelles comme des fibres de chanvre, des fibres de lin ou des fibres de soie, ou encore de mélanges de telles fibres, auquel cas ce renfort peut se présenter, selon la nature des fibres qui le constituent, sous la forme de fils coupés, de fibres broyées, de mats à filaments continus, de mats à filaments coupés, de stratifils (ou « rovings » en langue anglaise), de tissus, de tricots, de feutres, ou encore sous la forme de complexes réalisés par association de différents types de matériaux plans.

II peut également s'agir d'un renfort constitué de particules telles que des particules de liège ou des charges réfractaires du type tungstène, oxyde de magnésium, oxyde de calcium, alumine, silice, dioxyde de zirconium, dioxyde de titane, oxyde de béryllium.

Par ailleurs, la fabrication du matériau composite, et donc l'ajout de renfort dans la résine, peut être réalisée par toutes les techniques connues de l'homme du métier des matériaux composites comme, par exemple, par imprégnation, par moulage par injection simultanée, par moulage par drapage autoclavé, par moulage sous vide, par moulage par injection basse pression de résine (ou RTM pour « Resin Transfert Molding »), par moulage à la presse à froid "voie humide" basse pression, par moulage par injection de compound (ou BMC pour « Bulk Molding Compound »), par moulage par compression de mats préimprégnés (ou SMC pour « Sheet Molding Compound »), par enroulement filamentaire, par centrifugation ou encore par pultrusion, l'imprégnation étant préférée dans le cas où le renfort est constitué de fibres.

De préférence, le matériau composite est un matériau composite ablatif et, plus spécifiquement, un matériau composite ablatif de protection thermique, notamment pour l'aérospatiale.

De façon avantageuse, l'enthalpie de polymérisation de l'étape b) est inférieure à 500 J / g.

Avantageusement, le taux de coke de la résine thermodurcie obtenue à l'étape c) est supérieur à 50%.

La présente invention sera mieux comprise à la lecture de la description des exemples qui suivent qui sont donnés à titre indicatifs.

### Exemple 1 : Conversion du résorcinol propargylé et préparation de la résine selon l'invention

### Synthèse du résorcinol propargylé

10g (0,091 mol) de résorcinol (Alfa Aesar) est solubilisé dans 50 mL de diméthylsulfoxyde (DMSO), 50g (0,363 mol) de carbonate de potassium (K₂CO₃) est broyé puis ajouté sous agitation magnétique et le milieu est chauffé à 70°C (ext). 14,45 mL (2,2 eq.) de chlorure de propargyle (ABCR) est ajouté au goutte à goutte. La réaction est contrôlée par CCM avec un éluant 7:3 (volume) éther de pétrole : diéthyl éther. Après filtration et dilution dans 100 mL d'acétate d'éthyle le milieu est extrait avec 3x100 mL de saumure. La phase organique est séchée sur MgSO₄, filtrée et concentrée sous pression réduite. Le composé est purifié par distillation sous vide (T°C = 120°C, 4,5 Pa). Le rendement est de 77,4%.

### Conversion du résorcinol propargylé

Peser 0,0415 grammes (1%mol) de catalyseur (Acetonitrile)[(2-biphenyl)di-tert-butylphosphine]or(I) hexafluoroantimonate (ABCR) dans un pilulier sans précaution pour exclure l'air. 1 gramme (0,0054 mol) de résorcinol propargylé obtenu précédemment est introduit dans un tube de schlenk puis scellé. Le milieu est mis sous atmosphère inerte par vide/remplissage d'argon successif, au moins 3 fois. 50 mL de dichlorométhane (DCM) sec (purifié avec un dispositif PureSolv MD7) est ajouté à l'aide d'une seringue. Le catalyseur est introduit à contre-courant d'argon à l'aide du pilulier puis le milieu est conservé sous argon. La réaction est contrôlée par CCM avec un éluant 9:1 (volume) éther de pétrole : acétate d'éthyle. Après complétion de la réaction (durée : moins d'1 min), le milieu est filtré sur une fine couche de silice pour enlever le catalyseur. La proportion de fonctions éthers de propargyle résiduelles est estimée par RMN ¹H à 0%. Le rendement est de 65,8%. La proportion molaire de chromène de formule C dans le mélange est de 50%. La proportion molaire de chromène de formule D dans le mélange est de 50%. 6% de composé 2-methylbenzofuran sont présents dans le produit.

Le produit n'a pas été purifié d'avantage et est utilisé brut. La proportion de fonctions éthers de propargyle résiduelles inférieure à 11% est conforme au cahier des charges fixé.

### Polymérisation du chromène issu du résorcinol propargylé

La polymérisation des mélanges propargyle-chromène se fait par montée progressive en température. Dans le cas d'un mélange résorcinol propargyle-chromène avec une proportion en fonction propargyle résiduelle inférieure à 11% tel qu'obtenu précédemment, le traitement thermique appliqué est le suivant : 2h à 80°C, 2h à 100°C, 2h à 110°C, 2h à 120°C, 2h à 130°C et 2h à 150°C.

Un recuit à 220°C peut être effectué pour augmenter les propriétés thermo-mécaniques.

Le taux de coke obtenu avant recuit est de 63%.

### Exemple 2 : Conversion du résorcinol propargylé

Peser 0,0207 grammes (0,5%mol) de catalyseur (Acetonitrile)[(2-biphenyl)di-tert-butylphosphine]or(I) hexafluoroantimonate (ABCR) dans un pilulier sans précaution pour exclure l'air. 1 gramme (0,0054 mol) de résorcinol propargylé obtenu selon le procédé indiqué dans l'exemple 1 est introduit dans un tube de schlenk. 50 mL de dichlorométhane (DCM) est ajouté sans prendre de précaution afin d'exclure l'air. Le catalyseur est introduit à l'aide du pilulier sans prendre de précaution d'exclure l'air. La réaction est contrôlée par CCM avec un éluant 9:1 (volume) éther de pétrole : acétate d'éthyle. Après complétion de la réaction, le milieu est filtré sur une fine couche de silice pour enlever le catalyseur. La proportion de fonctions éthers de propargyle résiduelles est estimée par RMN ¹H à 8%. Le rendement est de 74%. 4,7% de composé 2-methylbenzofuran sont présents dans le produit. Le produit n'a pas été purifié d'avantage et est utilisé brut. La proportion de fonctions éthers de propargyle résiduelles inférieure à 11% est conforme au cahier des charges fixé.

### Exemple 3 : Conversion du résorcinol propargylé

Peser 0,0124 grammes (0,3%mol) de catalyseur (Acetonitrile)[(2-biphenyl)di-tert-butylphosphine]or(I) hexafluoroantimonate (ABCR) dans un pilulier sans précaution pour exclure l'air. 1 gramme (0,0054 mol) de résorcinol propargylé obtenu selon le procédé indiqué dans l'exemple 1 est introduit dans un tube de schlenk puis fermé. Le milieu est mis sous atmosphère inerte par vide/remplissage d'argon successif, au moins 3 fois. 10 mL de tétrahydrofurane (THF) sec (purifié avec un dispositif PureSolv MD7) est ajouté à l'aide d'une seringue. Le catalyseur est introduit à contre-courant d'argon à l'aide du pilulier puis le milieu est conservé sous argon. La réaction est contrôlée par CCM avec un éluant 9:1 (volume) éther de pétrole : acétate d'éthyle. Après complétion de la réaction, le milieu est filtré sur une fine couche de silice pour enlever le catalyseur. Cependant, celui-ci n'est pas retenu. La proportion de fonctions éthers de propargyle résiduelles est estimée par RMN ¹H à 0%. Le rendement est de 99,9%. La proportion molaire de chromène de formule C dans le mélange est de 50%. La proportion molaire de chromène de formule D dans le mélange est de 50%. 1,9% de composé 2-methylbenzofuran sont présents dans le produit. Le produit n'a pas été purifié d'avantage et est utilisé brut La proportion de fonctions éthers de propargyle résiduelles inférieure à 11% est conforme au cahier des charges fixé.

### Exemple 4 : Conversion du résorcinol propargylé

Peser 0,0124 grammes (0,3%mol) de catalyseur (Acetonitrile)[(2-biphenyl)di-tert-butylphosphine]or(I) hexafluoroantimonate (ABCR) dans un pilulier sans précaution pour exclure l'air. 1 gramme (0,0054 mol) de résorcinol propargylé obtenu selon le procédé indiqué dans l'exemple 1 est introduit dans un tube de schlenk puis fermé. Le milieu est mis sous atmosphère inerte par vide/remplissage d'argon successif, au moins 3 fois. 10 mL de méthyltétrahydrofurane (Me-THF) est ajouté à l'aide d'une seringue. Le catalyseur est introduit à contre-courant d'argon à l'aide du pilulier puis le milieu est conservé sous argon. La réaction est contrôlée par CCM avec un éluant 9:1 (volume) éther de pétrole : acétate d'éthyle. Après complétion de la réaction, le milieu est filtré sur une fine couche de silice pour enlever le catalyseur. Cependant, celui-ci n'est pas retenu. La proportion de fonctions éthers de propargyle résiduelles est estimée par RMN ¹H à 1,5%. Le rendement est de 86%. La proportion molaire de chromène de formule C dans le mélange est de 55%. La proportion molaire de chromène de formule D dans le mélange est de 45%. 7,1% de composé 2-methylbenzofuran sont présents dans le produit. Le produit n'a pas été purifié d'avantage et est utilisé brut. La proportion de fonctions éthers de propargyle résiduelles inférieure à 11% est conforme au cahier des charges fixé.

### Exemple 5 : Conversion du résorcinol propargylé

Peser 0,1244 grammes (0,3%mol) de catalyseur (Acetonitrile)[(2-biphenyl)di-tert-butylphosphine]or(I) hexafluoroantimonate (ABCR) dans un pilulier sans précaution pour exclure l'air. 10 grammes (0,054 mol) de résorcinol propargylé obtenu selon le procédé indiqué dans l'exemple 1 est introduit dans un tube de schlenk puis fermé. Le milieu est mis sous atmosphère inerte par vide/remplissage d'argon successif, au moins 3 fois. 100 mL de tétrahydrofurane (THF) sec (purifié avec un dispositif PureSolv MD7) est ajouté à l'aide d'une seringue. Le catalyseur est introduit à contre-courant d'argon à l'aide du pilulier puis le milieu est conservé sous argon et chauffé à 50°C. La réaction est contrôlée par CCM avec un éluant 9:1 (volume) éther de pétrole : acétate d'éthyle. Après 4h30 de réaction, le milieu est filtré sur une fine couche de silice pour enlever le catalyseur, Cependant, celui-ci n'est pas retenu. La proportion de fonctions éthers de propargyle résiduelles est estimée par RMN ¹H inférieure à 1%. Le rendement est supérieur à 97%. La proportion molaire de chromène de formule C dans le mélange est de 50%. La proportion molaire de chromène de formule D dans le mélange est de 50%. 3,3% de composé 2-methylbenzofuran sont présents dans le produit. Le produit n'a pas été purifié d'avantage et est utilisé brut. La proportion de fonctions éthers de propargyle résiduelles inférieure à 11% est conforme au cahier des charges fixé.

### Exemple 6 : Conversion de l'eugénol propargylé et préparation de la résine selon l'invention

### Synthèse de l'eugénol propargarlé

L"eugénol (Sigma Aldrich) (200g), le K₂CO₃ (211g) et le diméthylformamide (DMF) (2000mL) sont introduits dans un ballon de 6L et sont chauffés à 75°C sous agitation mécanique. Le chlorure de propargyle (ABCR) à 70% dans le toluène (158,5 mL) est ajouté goutte à goutte à l'aide d'une ampoule de coulée et le milieu réactionnel est chauffé et agité à 75°C pendant la nuit. La réaction est contrôlée par CCM avec un éluant éther de pétrole/éther diéthylique 7:3 (volume). Après réaction, le milieu réactionnel est filtré puis dilué et rincé à l'acétate d'éthyle. La phase organique est rincée à l'eau jusqu'à décoloration de la phase aqueuse (4 fois). La phase organique est séchée sur MgSO₄ et concentrée sous vide. Le rendement du brut est de 93%. Le composé est purifié par distillation sous vide (p=4,5 Pa et T°C = 60°C). Le rendement du composé distillé est de 90%.

### Conversion de l'eugénol propargylé

Peser 0,076 grammes (0,1%mol) de catalyseur (Acetonitrile)[(2-biphenyl)di-tert-butylphosphine]or(I) hexafluoroantimonate (ABCR) dans un pilulier sans précaution pour exclure l'air. 20 grammes (0,0054 mol) d'eugénol propargylé obtenu précédemment est introduit dans un ballon tricol de 250 mL puis scellé. Le milieu est mis sous atmosphère inerte par un léger flux d'argon. 100 mL de dichlorométhane sec (purifié avec un dispositif PureSolv MD7) est ajouté à l'aide d'une seringue. Le catalyseur est introduit à contre-courant d'argon à l'aide du pilulier puis le milieu est conservé sous argon et placé dans un bain d'huile préchauffé à 40°C. La réaction est contrôlée par CCM avec un éluant 9:1 (volume) éther de pétrole : acétate d'éthyle. Après 1 heure de réaction, le milieu est filtré sur une fine couche de silice pour enlever le catalyseur. La proportion de fonctions éthers de propargyle résiduelles est estimée par RMN ¹H inférieure à 1%. Le rendement est supérieur à 95%. 11% de composé 2-methylbenzofuran sont présents dans le produit. L'eugénol chromène est isolé par colonne chromatographie avec un éluant 9:1 (volume) éther de pétrole : acétate d'éthyle. Le rendement du produit pur est de 65%.

### Polymérisation du chromène issu de l'eugénol couplé propargylé

Le procédé mis en œuvre est identique à celui décrit dans l'exemple 1 pour le résorcinol propargylé à l'exception du fait que le traitement thermique appliqué est le suivant : 1h à 100°C, 1h30 à 150°C, 3h30 à 210°C et 1h à 200°C.

### Exemple 7 : Conversion de l'eugénol couplé propargylé et préparation de la résine selon l'invention

### Synthèse de l'eugénol couplé

26,675g (0,1625 mol) d'eugénol (Sigma Aldrich) est introduit dans un ballon tricol de 50 mL avec un agitateur magnétique. 0,1337g (0,1%mol) de catalyseur de Grubbs (Sigma Aldrich) de 1ère génération est introduit à l'aide d'un pilulier à contrecourant d'argon. Le milieu est directement mis sous vide poussé (3 kPa) et laissé agiter pendant 12h. Un spectre RMN ¹H du brut est effectué pour déterminer la conversion de l'eugénol. La conversion est de 67% molaire de composés eugénol couplé, les 37% molaire restant étant un mélange d'eugénol non réagit et de son isomère l'isoeugénol. La proportion stœchiométrique est évaluée par RMN ¹H à 34,3%/65,7% pour les composés eugénol couplé cis- et trans-respectivement. Le milieu est solubilisé dans un minimum d'éther à reflux, puis laissé reposer à température ambiante. Le solide est filtré sous pression réduite sur un fritté porosité 4, puis lavé avec 4x20mL de cyclohexane. Le solide est séché sous vide poussé à l'aide d'une pompe à palette pendant 10h. Afin de retirer le catalyseur de Grubbs 1ère génération, le solide est solubilisé dans le dichlorométhane (DCM) puis filtré sur célite. Un dépôt noir est observé sur la célite. La phase organique est séchée sous pressions réduite. Le rendement obtenu est de 25%.

### Synthèse de l'eugénol couplé propargylé

3g (0,010 mol) d'eugénol couplé obtenu précédemment est solubilisé dans 30 mL (10eqm) de DMF. 5,52g (4eq.) Du carbonate de potassium finement broyé (K₂CO₃) est ajouté sous agitation magnétique. 2,78 mL (2,5eq.) de bromure de propargyle (Alfa Aesar) (80%m dans le toluène) est ajouté à l'aide de l'ampoule de coulée. L'agitation magnétique est maintenue pendant 12h. La complétion de la réaction est contrôlée par CCM avec un éluant éther de pétrole : Acétate d'éthyle 50:50 (volume). Après filtration du K₂CO₃ et lavage au DMF, un excès d'eau distillée (200 mL) est ajouté pour faire précipiter le produit. Le solide est récupéré. 200 mL d'acétate d'éthyle est ajouté dans le milieu pour l'extraction. La phase aqueuse est jetée. Le solide récupéré préalablement est redissout dans la phase organique. La phase organique est lavée 3 fois à l'eau distillée (3x100 mL) et 1 fois à la saumure (1x100mL). La phase organique est séchée sur MgSO₄, filtrée et concentrée sous pression réduite. Le rendement est de 90%.

### Conversion de l'eugénol couplé propargylé

Peser 0,0308g (1%mol) de catalyseur (Acetonitrile)[(2-biphenyl)di-tert-butylphosphine]or(I) hexafluoroantimonate (ABCR) dans un pilulier sans précaution d'exclure l'air. Introduire 1,5g (0,0040 mol) d'eugénol couplé propargylé obtenu précédemment dans un tube de schlenk de 100 mL et faire des cycles vide/argon 3 fois. Puis ajouter 10 mL de dichlorométhane sec (purifié à l'aide d'un dispositif PureSolv MD7) à l'aide d'une seringue. Introduire le catalyseur à contre-courant d'argon puis sceller le milieu sous argon. Le contrôle de la réaction est effectué par CCM avec un éluant 9:1 (volume) Ether de pétrole : Acétate d'éthyle. Après 5 minutes de réaction, le milieu est filtré sur un fritté porosité 4 avec une courte couche de silice à l'aide du DCM. Le DCM est évaporé sous pression réduite à l'évaporateur rotatif. La conversion est de 100%. La proportion de fonctions éthers de propargyle résiduelles est estimée par RMN ¹H à 0%. Le rendement du brut est de 77,6%. Le brut de réaction est composé de 15% de composé 2-methylbenzofuran. Le produit est purifié par colonne chromatographie avec un éluant 9:1 (volume) éther de pétrole : acétate d'éthyle. L'eugénol couplé chromène pur est obtenu avec un rendement de 40%. La proportion de fonctions éthers de propargyle résiduelles inférieure à 39% est conforme au cahier des charges fixé.

### Polymérisation du chromène issu de l'eugénol couplé propargylé

Le procédé mis en œuvre est identique à celui décrit dans l'exemple 1 pour le résorcinol propargylé à l'exception du fait que le traitement thermique appliqué est le suivant : 1h à 80°C, 2h à 150°C, 2h à 220°C.

Le taux de coke obtenu avant recuit est de 56%.

### Exemple 8 : Conversion de l'isoeugénol couplé propargylé et préparation de la résine selon l'invention

### Synthèse de l'isoeugénol couplé

0,0181g (0,017%) de catalyseur de Grubbs II (UMICORE M2a) est introduit dans un ballon de 50 mL avec un agitateur magnétique, puis 20g (0,122 mol) d'isoeugénol (Sigma Aldrich) est ajouté. Le milieu est placé sous un flux d'argon et chauffé à 90°C. Le milieu devient solide après 3 minutes de réaction. Après refroidissement, un spectre RMN ¹H du brut est effectué pour déterminer la conversion de l'isoeugénol en stilbène. Le taux de conversion est de 90%. Uniquement le composé trans- est observé. Le produit est récupéré par suspension dans 4vol. de DCM, le milieu est chauffé à reflux pendant 1heure jusqu'à complète solubilisation puis laissé au repos à température ambiante toute la nuit. La suspension est filtrée sur fritté et lavée avec 1vol. de cyclohexane. Le rendement isolé est de 67%.

### Synthèse de l'isoeugénol couplé propargylé

10g (0,037 mol) d'eugénol stilbène obtenu précédemment est solubilisé dans 100mL (10eqm) de DMF. 25g (4,5eq.) Du carbonate de potassium finement broyé (K₂CO₃) est ajouté sous agitation magnétique. 10,23mL (2,5eq.) de bromure de propargyle (Alfa Aesar) (80%m dans le toluène) est ajouté à l'aide d'une seringue. L'agitation magnétique est maintenue pendant 12h. La complétion de la réaction est contrôlée par CCM avec un éluant éther de pétrole : Acétate d'éthyle 50:50 (volume). La conversion est totale après une nuit de réaction. Le K₂CO₃ est filtré puis lavé avec de l'acétate d'éthyle. Le composé est extrait avec 2x100 mL d'acétate d'éthyle. Les phases organiques sont lavées 4x100 mL à la saumure. Les phases organiques sont séchées sur MgSO₄, filtrées et concentrées sous pression réduite. Le rendement du produit est de 30%.

### Conversion de l'isoeugénol couplé propargylé

Peser 0,0054g (0,5%mol) de catalyseur (Acetonitrile)[(2-biphenyl)di-tert-butylphosphine]or(I) hexafluoroantimonate (ABCR) dans un pilulier sans précaution pour exclure l'air. Introduire 0,4864g (0,0014 mol) d'eugénol stilbène propargylé obtenu précédemment dans un tube de schlenk de 100 mL et faire des cycles vide/argon 3 fois. Puis ajouter 10 mL DCM sec (purifié avec un dispositif PureSolv MD7) à l'aide d'une seringue. Introduire le catalyseur à contrecourant d'argon. Ajouter un ballon de baudruche rempli d'argon. Le milieu est chauffé entre 30 et 40°C pour solubiliser totalement l'eugénol stilbène propargylé dans le DCM. La réaction est contrôlée par CCM avec éluant 5:5 (volume) éther de pétrole : acétate d'éthyle. Après réaction, le milieu est filtré sur un fritté porosité 4 avec une courte couche de silice à l'aide du DCM. Le DCM est évaporé sous pression réduite à l'évaporateur rotatif. La proportion de fonctions éthers de propargyle résiduelles est estimée par RMN ¹H inférieure à 1%. Le rendement du brut est de 56%. Le brut de réaction est composé de 14% de composé de type 2-methylbenzofuran estimé par RMN ¹H. Le produit n'a pas été purifié d'avantage et est utilisé brut. La proportion de fonctions éthers de propargyle résiduelles inférieure à 35% est conforme au cahier des charges fixé.

### Polymérisation du chromène issu de l'isoeugénol couplé propargylé

Le procédé mis en œuvre est identique à celui décrit dans l'exemple 1. L'enthalpie de réaction de 170 J/g.

### Exemple 9 : Conversion de l'isoeugénol propargylé et préparation de la résine selon l'invention

### Synthèse de l'isoeuqénol propargarlé

20g (0,130 mol) d'isoeugénol (Sigma Aldrich) est solubilisé dans 100 mL (5eqm) de DMF. 33,67g (2eq.) Du carbonate de potassium finement broyé (K₂CO₃) est ajouté sous agitation magnétique. 20,35 mL (1,5eq.) de bromure de propargyle (Alfa Aesar) (80%m dans le toluène) est ajouté à l'aide de l'ampoule de coulée. L'agitation magnétique est maintenue pendant 12h. La complétion de la réaction est contrôlée par CCM avec un éluant éther de pétrole : Acétate d'éthyle 70:30 (volume). Après filtration du K₂CO₃ et lavage à l'acétate d'éthyle, 100 mL d'acétate d'éthyle est ajouté dans le milieu pour l'extraction. La phase organique est lavée 3 fois à l'eau distillée (3x100 mL) et 1 fois à la saumure (1x100 mL). La phase organique est séchée sur MgSO₄, filtrée et concentrée sous pression réduite. Le rendement est de 91%.

Le composé est purifié par distillation sous vide au four à boule (p=15 Pa et T°C chauffage = 140°C). Le composé est récupéré sous forme de cristaux blancs. Le rendement global après purification est de 73%.

### Conversion de l'isoeugénol propargylé

Peser 0,3818g (0,0005 mol) de catalyseur (Acetonitrile)[(2-biphenyl)di-tert-butylphosphine]or(I) hexafluoroantimonate (ABCR) dans un pilulier sans précaution d'exclure l'air. Introduire 10g (0,05 mol) d'isoeugénol propargylé obtenu précédemment dans un ballon tricol de 100 mL surmonté d'un réfrigérant. Le milieu est mis sous argon par une des entrées du tricol. 40 mL de DCM sec (purifié par un dispositif PureSolv MD7) est ajouté à l'aide d'une seringue. Le catalyseur est introduit à contrecourant d'argon à l'aide du pilulier par la seconde entrée du tricol et le milieu est conservé sous argon. La réaction est contrôlée par CCM avec éluant 9:1 (volume) Ether de pétrole : Acétate d'éthyle. Après 5 minutes, la conversion est totale, la proportion de composé 2-methylbenzofuran est estimée par RMN ¹H à 13,6%. Le milieu est concentré sous pression réduite puis purifié par colonne chromatographie par dépôt solide avec un éluant 9:1 (volume) Ether de pétrole : Acétate d'éthyle. Le rendement d'isoeugénol chromène est de 69%.

Il est possible de diminuer la quantité de 2-methylbenzofuran formé en effectuant la réaction à basse température (0°C). Après 2 heures, la conversion est totale, la proportion de composé 2-methylbenzofuran est estimée par RMN ¹H à 4,2%.

### Polymérisation du chromène issu de l'isoeugénol propargylé

Le procédé mis en œuvre est identique à celui décrit dans l'exemple 1.

## Revendications

1. Procédé de préparation d'un matériau en résine thermodurcie comprenant les étapes successives suivantes :
- a) transformation d'un éther de propargyle aromatique de formule générale (I) suivante 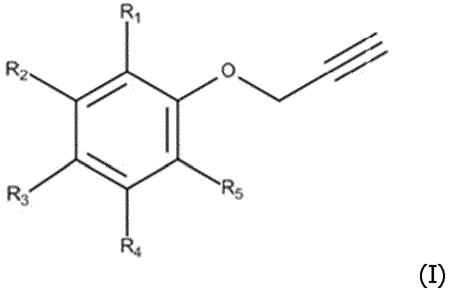 dans laquelle :
**R₁ et R₅** représentent indépendamment l'un de l'autre un atome d'hydrogène, un groupe alcène en C₂-C₆, alcyne en C₂-C₆, O-alkyle en C₁-C₆, O-alcène en C₂-C₆ ou O-alcyne en C₂-C₆, à la condition que l'un au moins des **R₁ et R₅** représente un atome d'hydrogène et que les groupes **R₁ et R₅** ne représentent pas un groupe O-propargyl ;
**R₂ et R₄** représentent indépendamment l'un de l'autre un atome d'hydrogène, un groupe alcène en C₂-C₆, alcyne en C₂-C₆ tel qu'un propargyle, O-alkyle en C₁-C₆, O-alcène en C₂-C₆ ou O-alcyne en C₂-C₆ tel qu'un O-propargyle ;
**et R₃** représente un atome d'hydrogène ou un groupe alcène en C₂-C₆, le groupe alcène étant éventuellement substitué par un groupe de formule générale (II) suivante 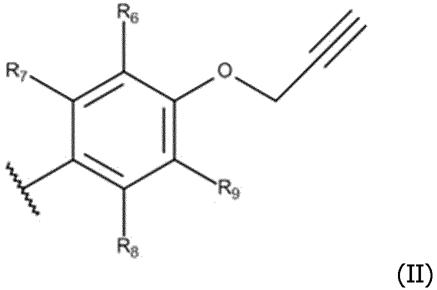 dans laquelle :
**R₆ et R₉** représentent indépendamment l'un de l'autre un atome d'hydrogène, un groupe alcène en C₂-C₆, alcyne en C₂-C₆, O-alkyle en C₁-C₆, O-alcène en C₂-C₆ ou O-alcyne en C₂-C₆, à la condition que l'un au moins des **R₆ et R₉** représente un atome d'hydrogène ;
et **R₇ et R₈** représentent indépendamment l'un de l'autre un atome d'hydrogène, un groupe alcène en C₂-C₆, alcyne en C₂-C₆ tel qu'un propargyle, O-alkyle en C₁-C₆, O-alcène en C₂-C₆ ou O-alcyne en C₂-C₆ tel qu' un O-propargyle ;
à la condition que l'un au moins des **R₁, R₂, R₃, R₄ et R₅** ne représente pas un atome d'hydrogène ou un groupe O-alkyle en C₁-C₆ ;
et ses isomères cis/trans et ses isomères optiques et leurs mélanges racémiques
en un chromène par catalyse homogène à l'or (I) avec le catalyseur (acétonitrile)[(2-biphényl)di-tert-butylphosphine]or(I) hexafluoroantimonate dans un solvant organique sous atmosphère inerte ou non inerte ;
- b) polymérisation du produit de la réaction obtenu à l'étape a) de façon à obtenir le matériau en résine thermodurcie ;
- c) récupération du matériau en résine thermodurcie obtenu à l'étape b).

2. Procédé selon la revendication 1, **caractérisé en ce que** l'enthalpie de polymérisation de l'étape b) est inférieure à 500 J / g.

3. Procédé selon l'une quelconque des revendications 1 ou 2, **caractérisé en ce que** le taux de coke de la résine thermodurcie obtenue à l'étape c) est supérieur à 50%.

4. Procédé selon l'une quelconque des revendications 1 à 3, **caractérisé en ce que** l'éther de propargyle aromatique de formule générale (I) est choisi dans le groupe constitué par le résorcinol propargylé, l'eugénol propargylé, l'eugénol couplé propargylé, l'isoeugénol couplé propargylé, l'isoeugénol propargylé et leurs mélanges et leurs isomères cis/trans et leurs isomères optiques et leurs mélanges racémiques, avantageusement il s'agit du résorcinol propargylé.

5. Procédé selon la revendication 4, **caractérisé en ce que** le pourcentage molaire de fonctions propargyles résiduelles du chromène est inférieur à 11% lorsque l'éther de propargyle aromatique de formule générale (I) est le résorcinol propargylé, le pourcentage molaire de fonctions propargyles résiduelles du chromène est inférieur à 39% lorsque l'éther de propargyle aromatique de formule générale (I) est l'eugénol couplé propargylé et le pourcentage molaire de fonctions propargyles résiduelles du chromène est inférieur à 35% lorsque l'éther de propargyle aromatique de formule générale (I) est l'isoeugénol couplé propargylé.

6. Procédé selon l'une quelconque des revendications 1 à 5, **caractérisé en ce que** le solvant organique de l'étape a) est choisi parmi le tétrahydrofurane et le 2-méthyltétrahydrofurane.

7. Procédé selon l'une quelconque des revendications 1 à 6, **caractérisé en ce que** la teneur en catalyseur de l'étape a) dans le milieu réactionnel est comprise entre 0,1 et 2% molaire, avantageusement entre 0,1 et 0,5 % molaire.

8. Procédé selon l'une quelconque des revendications 4 à 7, **caractérisé en ce que** l'éther de propargyle aromatique de formule générale (I) est le résorcinol propargylé et **en ce que** le chromène obtenu à l'étape a) a la formule C et / ou D suivante, avantageusement est un mélange des formules C et D

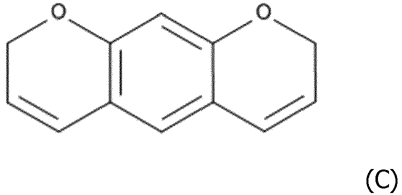

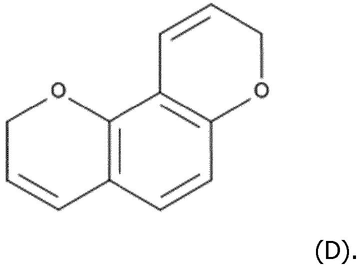

9. Procédé selon l'une quelconque des revendications 1 à 8, **caractérisé en ce que** le catalyseur est éliminé du milieu réactionnel à la fin de l'étape a).

## Patentansprüche

1. Verfahren zum Herstellen eines Materials aus wärmegehärtetem Harz, umfassend die folgenden aufeinanderfolgenden Schritte:
a) Umwandeln eines aromatischen Propargylethers der folgenden allgemeinen Formel (I) 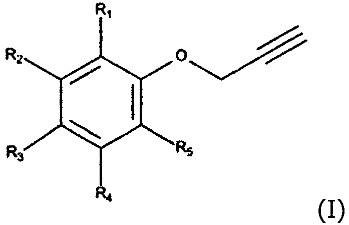 wobei:
R₁ und R₅ unabhängig voneinander Wasserstoff, C₂-C₆-Alken, C₂-C₆-Alkin, O-C₁-C₆-Alkyl, O-C₂-C₆-Alken oder O-C₂-C₆-Alkin darstellen, mit der Maßgabe, dass mindestens eines von R₁ und R₅ ein Wasserstoffatom darstellt und dass die Gruppen R₁ und R₅ keine Gruppe O-Propargyl darstellen;
R₂ und R₄ unabhängig voneinander Wasserstoff, C₂-C₆-Alken, C₂-C₆-Alkin, wie beispielsweise Propargyl, O-C₁-C₆-Alkyl, O-C₂-C₆-Alken oder O-C₂-C₆-Alkin, wie beispielsweise O-Propargyl darstellen;
und R₃ ein Wasserstoffatom oder eine C₂-C₆-Alkengruppe darstellt, wobei die Alkengruppe optional mit einer Gruppe von folgender allgemeiner Formel (II) substituiert ist 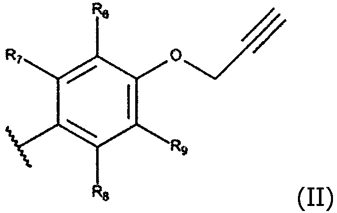 wobei:
R₆ und R₉ unabhängig voneinander Wasserstoff, C₂-C₆-Alken, C₂-C₆-Alkin, O-C₁-C₆-Alkyl, O-C₂-C₆-Alken oder O-C₂-C₆-Alkin darstellen, mit der Maßgabe, dass mindestens eines von R₆ und R₉ ein Wasserstoffatom darstellt;
und R₇ und R₈ unabhängig voneinander Wasserstoff, C₂-C₆-Alken, C₂-C₆-Alkin, wie beispielsweise Propargyl, O-C₁-C₆-Alkyl, O-C₂-C₆-Alken oder O-C₂-C₆-Alkin, wie beispielsweise O-Propargyl darstellen;
mit der Maßgabe, dass mindestens eines von R₁, R₂, R₃, R₄ und R₅ kein Wasserstoffatom oder eine O-C₁-C₆-Alkylgruppe darstellt;
und seine cis/trans-Isomere und seine optischen Isomere und racemischen Gemische zu einem Chromen durch homogene Gold(I)-Katalyse mit dem Katalysator (Acetonitril)[(2-biphenyl)di-tert-butylphosphin]or(I)hexafluoroantimonat in einem organischen Lösungsmittel unter einer inerten oder nicht inerten Atmosphäre;
b) Polymerisieren des in Schritt a) erlangten Reaktionsprodukts, um das wärmegehärtete Harzmaterial zu erlangen;
c) Rückgewinnen des in Schritt b) erlangten wärmegehärteten Harzmaterials.

2. Verfahren nach Anspruch 1, **dadurch gekennzeichnet, dass** die Polymerisationsenthalpie von Schritt b) weniger als 500 J/g ist.

3. Verfahren nach einem der Ansprüche 1 oder 2,
**dadurch gekennzeichnet, dass** der Koksgehalt des in Schritt c) erlangten wärmegehärteten Harzes mehr als 50 % ist.

4. Verfahren nach einem der Ansprüche 1 bis 3, **dadurch gekennzeichnet, dass** der aromatische Propargylether der allgemeinen Formel (I) ausgewählt ist aus der Gruppe, bestehend aus propargyliertem Resorcin, propargyliertem Eugenol, propargyliertem gekoppeltem Eugenol, propargyliertem gekoppeltem Isoeugenol, propargyliertem Isoeugenol und deren Gemischen und deren cis/trans-Isomeren und deren optischen Isomeren und deren racemischen Gemischen, vorzugsweise wobei es sich um propargyliertes Resorcinol handelt.

5. Verfahren nach Anspruch 4, **dadurch gekennzeichnet, dass** der molare Prozentsatz an restlichen Propargylfunktionen des Chromens weniger als 11 % ist, wenn der aromatische Propargylether der allgemeinen Formel (I) propargyliertes Resorcin ist, der Molprozentsatz an restlichen Propargylfunktionen des Chromens weniger als 39 % ist, wenn der aromatische Propargyläther der allgemeinen Formel (I) propargyliertes gekoppeltes Eugenol ist, und der Molprozentsatz an restlichen Propargylfunktionen des Chromens weniger als 35 % ist, wenn der aromatische Propargyläther der allgemeinen Formel (I) propargyliertes gekoppeltes Isoeugenol ist.

6. Verfahren nach einem der Ansprüche 1 bis 5, **dadurch gekennzeichnet, dass** das organische Lösungsmittel in Schritt a) ausgewählt ist aus Tetrahydrofuran und 2-Methyltetrahydrofuran.

7. Verfahren nach einem der Ansprüche 1 bis 6, **dadurch gekennzeichnet, dass** der Gehalt an Katalysator von Schritt a) in dem Reaktionsmedium zwischen 0,1 und 2 Mol-%, vorteilhafterweise zwischen 0,1 und 0,5 Mol-%, ist.

8. Verfahren nach einem der Ansprüche 4 bis 7, **dadurch gekennzeichnet, dass** der aromatische Propargylether der allgemeinen Formel (I) propargyliertes Resorcin ist und dass das in Schritt a) erlangte Chromen die folgende Formel C und/oder D aufweist, vorteilhafterweise ein Mischung der Formeln C und D

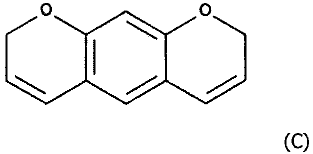

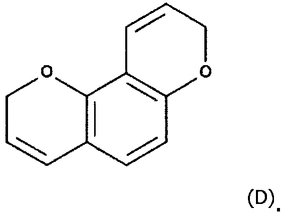

9. Verfahren nach einem der Ansprüche 1 bis 8,
**dadurch gekennzeichnet, dass** der Katalysator am Ende von Schritt a) aus dem Reaktionsmedium entfernt wird.

## Claims

1. Process for preparing a material made of thermoset resin, comprising the following successive steps:
- a) transforming an aromatic propargyl ether of general formula (I) below 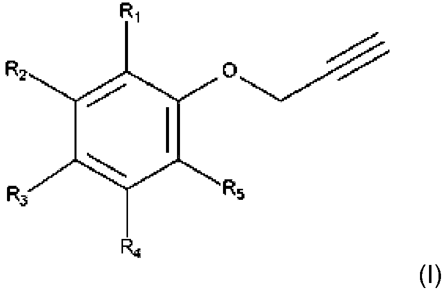 in which:
**R₁ and R₅** represent, independently of each other, a hydrogen atom, a C₂-C₆ alkene, C₂-C₆ alkyne, O-(C₁-C₆)alkyl, O-(C₂-C₆)alkene or O-(C₂-C₆)alkyne group, on condition that at least one from among **R₁ and R₅** represents a hydrogen atom and that the groups **R₁ and R₅** do not represent an O-propargyl group;
**R₂ and R₄** represent, independently of each other, a hydrogen atom, a C₂-C₆ alkene, C₂-C₆ alkyne such as propargyl, O-(C₁-C₆)alkyl, O-(C₂-C₆)alkene or O-(C₂-C₆)alkyne such as an O-propargyl;
and **R₃** represents a hydrogen atom or a C₂-C₆ alkene group, the alkene group being optionally substituted with a group of general formula (II) below 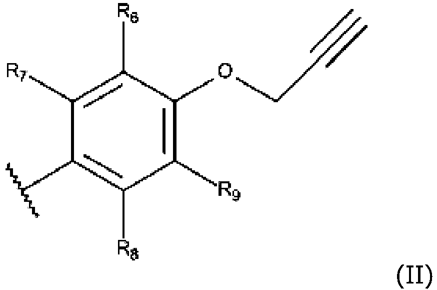 in which:
**R₆ and R₉** represent, independently of each other, a hydrogen atom, a C₂-C₆ alkene, C₂-C₆ alkyne, O-(C₁-C₆)alkyl, O-(C₂-C₆)alkene or O-(C₂-C₆)alkyne group, on condition that at least one from among **R₆ and R₉** represents a hydrogen atom;
and **R₇ and R₈** represent, independently of each other, a hydrogen atom, a C₂-C₆ alkene, C₂-C₆ alkyne such as propargyl, O-(C₁-C₆)alkyl, O-(C₂-C₆)alkene or O-(C₂-C₆)alkyne such as an O-propargyl;
on condition that at least one from among **R₁, R₂, R₃, R₄ and R₅** does not represent a hydrogen atom or a O-(C₁-C₆)alkyl group;
and the cis/trans isomers thereof and the optical isomers thereof and the racemic mixtures thereof
into a chromene by homogeneous gold(I) catalysis with the catalyst (acetonitrile)[(2-biphenyl)di-tert-butylphosphine]gold(I) hexafluoroantimonate in an organic solvent under an inert or non-inert atmosphere;
- b) polymerization of the reaction product obtained in step a) so as to obtain the material made of thermoset resin;
- c) recovery of the material made of thermoset resin obtained in step b).

2. Process according to Claim 1, **characterized in that** the enthalpy of polymerization of step b) is less than 500 J/g.

3. Process according to either of Claims 1 and 2, **characterized in that** the coke content of the thermoset resin obtained in step c) is greater than 50%.

4. Process according to any one of Claims 1 to 3, **characterized in that** the aromatic propargyl ether of general formula (I) is chosen from the group consisting of propargylated resorcinol, propargylated eugenol, propargylated coupled eugenol, propargylated coupled isoeugenol, propargylated isoeugenol and mixtures thereof and the cis/trans isomers thereof and the optical isomers thereof and the racemic mixtures thereof; advantageously, it is propargylated resorcinol.

5. Process according to Claim 4, **characterized in that** the molar percentage of residual propargyl functions in the chromene is less than 11% when the aromatic propargyl ether of general formula (I) is propargylated resorcinol, the molar percentage of residual propargyl functions in the chromene is less than 39% when the aromatic propargyl ether of general formula (I) is propargylated coupled eugenol and the molar percentage of residual propargyl functions in the chromene is less than 35% when the aromatic propargyl ether of general formula (I) is propargylated coupled isoeugenol.

6. Process according to any one of Claims 1 to 5, **characterized in that** the organic solvent of step a) is chosen from tetrahydrofuran and 2-methyltetrahydrofuran.

7. Process according to any one of Claims 1 to 6, **characterized in that** the content of catalyst of step a) in the reaction medium is between 0.1 mol% and 2 mol%, advantageously between 0.1 mol% and 0.5 mol%.

8. Process according to any one of Claims 4 to 7, **characterized in that** the aromatic propargyl ether of general formula (I) is propargylated resorcinol and **in that** the chromene obtained in step a) has the formula C and/or D below; advantageously, it is a mixture of formulae C and D

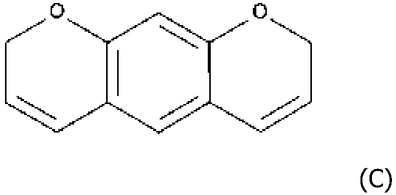

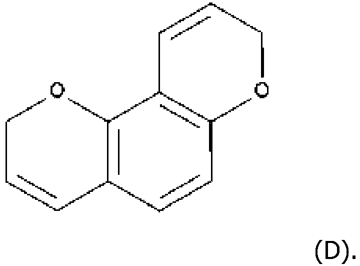

9. Process according to any one of Claims 1 to 8, **characterized in that** the catalyst is removed from the reaction medium at the end of step a).
